# EUROPEAN PATENT SPECIFICATION

(11) **EP 0 373 564 B1**
(45) Date of publication and mention of the grant of the patent: **19.05.1993**
(21) Application number: 89122846.2
(22) Date of filing: 11.12.1989
(51) Int. Cl.: A61M 5/175

(54) **Device for transforming a laminar fluid flow into drops**
Vorrichtung zur Umwandlung einer laminaren Strömung in Tropfen
Dispositif pour transformer un écoulement laminaire en gouttes

(30) Priority: 14.12.1988 YU 2260/88
(43) Date of publication of application: 20.06.1990
(73) Proprietor: Rajster, Vojko, YU-61230 Domzale (YU); Rajster, Ales, YU-61000 Ljubljana (YU)
(72) Inventor: Rajster, Vojko, YU-61230 Domzale (YU); Rajster, Ales, YU-61000 Ljubljana (YU)
(74) Representative: Lewald, Dietrich, Dipl.-Ing.

(56) References cited:
- CH-A- 519 658
- FR-A- 2 109 384
- FR-A- 2 227 019
- FR-A- 2 390 174
- FR-A- 2 468 049
- US-A- 3 398 860

## Description

The invention relates to a device for metering a fluid flow, especially of infusion fluid, consisting of an oblong cylindrical casing having, at one end, a supply pipe connection and, at the other end, a discharge pipe connection, and a throttling member, located within the cylindrical casing, adapted to adjust the length of a screw-shaped channel, which is also formed within said cylindrical casing.

A device with the above mentioned features is known from the patent specification US-A-3 398 860. Said specification discloses a metering device for liquids which is characterised by the feature that a helical capillary channel is provided in the internal surface of the tubular body or in the external surface of the pin. Said pin, the threaded part of which is screwable into a threaded bore of the connecting nozzle, is guided in a sealing ring inserted in the end part of the body thereby closing the bore of the latter. However, said device has serious drawbacks. Namely, in the case when it is desirable to make the adjustment of fluid flow as rapidly as possible, the pin must be moved into the direction averted from the nozzle. This causes rapid pressure drop in the chamber beneath said pin, which results in suction blood from the vein of the patient into the infusion system. Since needles having relatively small cross-sectional area are used during infusion, particularly in the case of infants, this could lead to clogging thereof.

Additionaly, a device for metering a fluid flow is known from patent specification FR-A-2 109 384, the solution practically having the same disadvantages as the device known from the US-A-3 398 860.

The object of the present invention is to eliminate the above discussed drawbacks of the prior art. This object is achieved by providing a metering device having the characteristics defined in claim 1.

According to the invention, the object is surprisingly achieved by means of cylindrical casing including an inlet unit, comprising a socket, which carries supply pipe connection and a thread on its external surface, a first cylinder concentrically connected through a first flange to said socket and a closed bottom. A screw-shaped channel is formed on the outside of the first cylinder and extends, towards the other end of the casing, from a through-hole which leads from the inside to the outside of the inlet unit perpendicularly to the wall of the first cylinder.

The metering device according to the invention further includes an intermediate unit comprising a second cylinder, a funnel-shaped part and a connecting second flange, perpendicular to the cylinder, a step being formed, with a number of longitudinally and radially running grooves, on a part of the cylinder internal surface close to the second flange and on the second flange itself.

The device has a levelling nut comprising a threaded collar screwed on said socket and integrally connected to said throttling member, which consists of a cylindrical sealing screen. The inlet unit is concentrically mounted into the intermediate unit by slip-fitting the first cylinder within the grooved step, whereby a cylindrical clearance is maintained between the screw-shaped channel and the wall of said first cylinder, as well as a distance is maintained between the funnel-shaped part and bottom of the inlet unit, due to the abutting of a shoulder of the second cylinder on said grooved second flange, and whereby said sealing screen is adapted to slip-fit into said clearance in order to progressively close said screw-shaped channel with the axial progression of said levelling nut so that the lenght of the closed part of said channel can be controlled by screwing or unscrewing said leveling nut.

According to the invention, the fit between the screen of the nut and the cylinder of the inlet unit is tighter than the fit between the sealing screen and the cylinder of the intermediate unit.

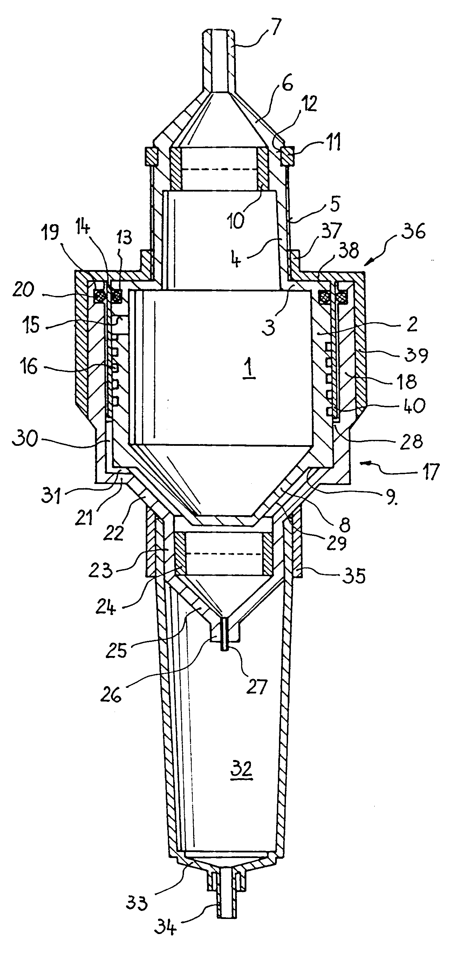

The invention will now be disclosed on the basis of an example of embodiment with reference to the accompanying drawing, which represents a longitudinal cross-section of the device according to the invention in a closed position.

The device according to the invention comprises an inlet unit 1 consisting of a cylinder 2 having, at its first end, a flange 3 running radially inwards with a socket 4 arranged in the longitudinal direction of the device and provided with an external thread 5 on its entire length. The socket 4 comprises a funnel-shaped extension 6 provided with a supply pipe connection 7. The cylinder 2 has a bottom 8 at its second end, a step 9 being arranged in the area where the bottom 8 passes into the cylinder 2.

Inside the socket 4 there is a fluid strainer 10 placed under the funnel-shaped extension 6. At the outer side of the socket 4 in the area where said socket passes into the extension 6 there is provided a stop ring 11 placed into a corresponding groove 12 on the socket 4. The cylinder 2 has a radially running groove 13 formed on its outside circumference, in which there is placed a seal ring 14. In the area below the seal ring 14 at the side turned towards the bottom 8, there is provided a through-hole 15 leading perpendicularly to the cylinder 2. From the latter towards the bottom 8 there is provided a screw-shaped channel 16 on the outside circumference of the cylinder 2, reaching into the area where the cylinder 2 passes into the bottom 8.

Furthermore, the device according to the invention comprises an intermediate unit 17 consisting of a cylinder 18 having a radially running groove 19 at its upper end in the area of its free edge and from inside, in which there is placed a seal ring 20.

At its lower end, the cylinder 18 has a flange 21 turned perpendicularly inwards, comprising a funnel-shaped part 22, which changes over into a socket 23 having a fluid strainer 24. The socket 23 ends with a funnel-shaped part 25 comprising a socket 26, wherein there is tightly arranged a dropping tube 27. The cylinder 18 of the unit 17 is, at its inner side and in the area of the flange 21, formed with a step 28 projecting radially inwards, which serves as a spacer between the cylinder 18 of the unit 17 and the inlet unit 1 tightly arranged therein; its cylinder 2 is integral with the flange 21 of the unit 17 by means of a step 9. Moreover, each time the distance 29 is formed between the bottom 8 of the unit 1 and the funnel-shaped part 22 of the unit 17. The step 28 comprises a number of longitudinally running grooves 30 on its inner circumference connected onto a number of radially running grooves 31, which are arranged on the flange 21.

Onto the socket 23 there is tightly slipped-on a conical collector 32 which has a bottom 33 with a discharge pipe connection 34 at its end that is averted from the socket 23. The collector 32 is protected against slipping off the socket 23 by means of a thrust ring 35.

Onto the socket 4 of the unit 1 having a thread 5 there is screwed on a levelling nut 36 comprising a thread collar 37 and a radially outwards running plane segment 38, whichs originates from the collar 37 and comprises a mantle 39 running perpendicularly thereto and close to the cylinder 18 of the unit 17. In the direction radially inwards from the mantle 39, the levelling nut 36 has a cylindric sealing screen 40 running in the longitudinal direction of the device; when the device according to the invention is assembled said screen is placed with transition fit between the cylinder 2 of the unit 1 and the cylinder 18 of the unit 17, i.e. into the clearance provided between the cylinder 2 and the cylinder 18 by means of the step 28. Moreover, the fit between the screen 40 and the cylinder 2 is tighter than the fit between the screen 40 and the cylinder 18, and the screen 40 itself in its closed position stretches in the longitudinal direction into the area of the step 28 whereby the channel 16 is completely overlapped.

The device according to the invention operates in the following manner.

The fluid flows inside the unit 1 through the supply pipe connection 7 and the fluid strainer 10. In the closed position shown in the drawing, the sealing screen 40 overlaps the screw-shaped channel 16 over its entire length and the flow is prevented. When unscrewing the levelling nut 36, the latter moves towards the stop ring 11, the channel 16 becomes free and the fluid begins to flow through the hole 15. The fluid glides through the channel 16 due to capillarity and continues its way in the grooves 30, 31, through the strainer 24 and the dropping tube 27 into the collector 32 and out of it through the discharge pipe connection 34.

The amount of the fluid flowing out and the number of drops per unit of time, respectively, are controlled by means of rotating of the nut 36, whereby a longer or a shorter screw-shaped channel 16 and, consequently, an accurate predetermined number of drops is achieved.

To achieve the optimum gliding of the fluid, the channel 16 is dimensioned in such a manner that its entire length to its free-space sectional area for the flow of e.g. 1.5 ml/h of the infusion fluid is in the proportion of 56000:1 for 5% solution, 40000:1 for 10% solution, 30000:1 for 20% solution and 25000:1 for 40% solution. It became evident when testing blood that for the same amount, i.e. 1.5 ml/h, the entire length of the channel 16 to the free-space sectional area is in the proportion of 22000:1.

## Claims

1. A device for metering a fluid flow, especially of infusion fluid, consisting of:
- an oblong cylindrical casing having, at one end, a supply pipe connection (7) and, at the other end, a discharge pipe connection (26), and
- a throttling member (40), located within the cylindrical casing, adapted to adjust the lenght of a screw-shaped channel (16), which is also formed within said cylindrical casing,
*characterized in that*
said cylindrical casing includes:
a) an inlet unit (1), comprising a socket (4), which carries said supply pipe connection and a thread (5) on its external surface, a first cylinder (2) concentrically connected through a first flange (3) to said socket (4) and a closed bottom (8), said screw-shaped channel (16) being formed on the outside of the first cylinder (2) and extending, towards the other end of the casing, from a through-hole (15) which leads from the inside to the outside of the inlet unit perpendicularly to the first cylinder's wall,
b) an intermediate unit (17) comprising a second cylinder (18), a funnel-shaped part (22) and a connecting second flange (21), perpendicular to the cylinder, a step (28) being formed, with a number of longitudinally and radially running grooves (30, 31), on a part of the cylinder's internal surface close to the second flange and on the second flange itself,
c) a levelling nut (36) comprising a threaded collar (37) screwed on said socket (4) and integrally connected to said throttling memeber, which consists of a cylindrical sealing screen (40),
the inlet unit being concentrically mounted into the intermediate unit by slipfitting the first cylinder (2) within the grooved step (28), whereby a cylindrical clearance is maintained between the screw-shaped channel (16) and the wall of said first cylinder (2), as well as a distance (29) is maintained between the funnel-shaped part (22) and bottom (8) of the inlet unit, due to the abutting of a shoulder (9) of the second cylinder on said grooved second flange (21), and whereby said sealing screen (40) is adapted to slip-fit into said clearance in order to progressively close said screw-shaped channel (40) with the axial progression of said levelling nut (36), so that the lenght of the closed part of said channel can be controlled by screwing or unscrewing said leveling nut.

2. Device according to claim 1, *characterized in that* the fit between the screen (40) of the nut (36) and the cylinder (2) of the inlet unit (1) is tighter than the fit between the screen (40) and the cylinder (18) of the intermediate unit (17).

## Patentansprüche

1. Vorrichtung zur Messung von Flüssigkeitsströmung, insbesondere von Infusionsflüssigkeit, bestehend aus:
- einem langlichen zylindrischen Gehäuse, das einen Zuführungsrohranschluss (7) an einem Ende und einen Abflussrohranschluss (34) am anderen Ende aufweist, und
- einem im Inneren des zylindrischen Gehäuses angeordneten Drosselorgan (40), das für Längennachstellen eines im Inneren des zylindrischen Gehäuses ausgebildeten schraubenförmigen Kanals (16) bestimmt ist,
*dadurch gekennzeichnet,*
dass ein zylindrisches Gehäuse einschliesst:
**a)** ein Zulaufelement (1) mit einem Rohransatz (4), welcher an seiner aussenseitigen Oberfläche den erwähnten Zuführungsrohranschluss und ein Gewinde (5) trägt, einem ersten Zylinder (2), der an einen geschlossenen Boden (8) und mittels einem ersten Flansch (3) an den erwähnten Rohransatz (4) konzentrisch angeschlossen ist, wobei der schraubenförmige Kanal (16) an der Aussenseite des ersten Zylinders (2) ausgebildet ist und sich von einer Durchbohrung (15), welche von der Innenseite bis zur Aussenseite des Zulaufelements und senkrecht auf die Wand des ersten Zylinders führt, gegen das andere Ende des Gehäuses erstreckt,
**b)** ein Zwischenelement (17) mit einem zweiten Zylinder (18), einem trichteiförmigen Teil (22) und einem auf dem Zylinder senkrecht stehenden, verknüpfenden zweiten Flansch (21), wobei an einem Teil der innenseitigen Oberfläche des Zylinders, nahebei dem zweiten Flansch und am zweiten Flansch selbst, eine Stufe (28) mit einer Reihe von längs- und radialverlaufenden Rillen (30,31) ausgebildet ist,
**c)** eine Stellmutter (36) mit einem Gewindebund (37), der an den erwähnten Rohransatz (4) angeschraubt und einteilig mit dem erwähnten Drosselorgan, das aus einer zylindrischen Dichtungswand (40) besteht, verbunden ist,
dass das Zulaufelement durch eine Spielpassung des ersten Zylinders (2) und der gerillten Stufe (28) in dem Zwischenelement konzentrish angeordnet ist, wodurch ein zylindrischer Abstand zwischen dem schraubenförmigen Kanal (16) und der Wand des erwähnten ersten Zylinders (2) als auch ein Zwischenraum (29) zwischen dem trichterförmigen Teil (22) und dem Boden (8) des Zulaufelements wegen Anliegen der Schulter (9) des zweiten Zylinders an den erwähnten gerillten zweiten Flansch (21) gehalten ist, und wobei die erwähnte Dichtungswand (40) derart ausgebildet ist, dass sie in den erwähnten Zwischenraum hineinschlüpft, um den schraubenförmigen Kanal (16) durch die fortschreitende Bewegung der erwähnten Stellmutter (36) fliessend verschliessen zu können, so dass die Länge des verschlossenen Teils des erwähnten Kanals durch Ein- oder Abschrauben der erwähnten Stellmutter geregelt wird.

2. Vorrichtung nach Anspruch 1, *dadurch gekennzeichnet,* dass die Passung zwischen der Dichtungswand (40) der Stellmutter (36) und dem Zylinder (2) des Zulaufelements (1) enger ausgebildet ist als die Passung zwischen der Dichtungswand (40) und dem Zylinder (18) des Zwischenelements (17).

## Revendications

1. Dispositif de contrôle d'un débit de liquide (et en particulier d'un liquide à perfuser) constitué:
- d'un boîtier cylindrique allongé possédant à une extrémité un raccord (7) pour tuyau d'alimentation et à l'autre extrémité un raccord (26) pour tuyau je décharge, et
- d'un élément d'étranglement (40) situé dans le boîtier cylindrique, adapté pour régler la longueur d'un canal de forme hélicoïdale (16) également formé dans ledit boîtier cylindrique,
**caractérisé en ce que** ledit boîtier cylindrique comprend :
a) une unité d'entrée (1) comprenant une douille (4) portant ledit raccord de tuyau d'alimentation et un filet (5) à sa surface extérieure, un premier cylindre (2) relié concentriquement par l'intermédiaire d'une première bride (3) à ladite douille (4) et un fond fermé (8), ledit canal de forme hélicoïdale (16) étant formé à l'extérieur du premier cylindre (2) et s'étendant en direction de l'autre extrémité du boîtier entre un orifice traversant (15) reliant l'intérieur à l'extérieur de l'unité d'entrée perpendiculairement à la première paroi du cylindre,
b) une unité intermédiaire (17) comprenant un second cylindre (18), une partie (22) en forme d'entonnoir et une seconde bride de liaison (21) perpendiculaire au cylindre, un épaulement (28) portant plusieurs sillons (30, 31) s'étendent longitudinalement et radialement étant formé sur une partie de la surface inférieure du cylindre à proximité de la seconde bride et sur la seconde bride elle-même,
c) un écrou de réglage (36) comprenant un collier fileté (37) vissé sur ladite douille (4) et faisant partie intégrante dudit élément d'étranglement constitué d'un écran hermétique cylindrique (40),
l'unité étant montée concentriquement dans l'unité intermédiaire en ajustant par glissement le premier cylindre (2) dans l'épaulement rainuré (28), un eu cylindrique étant conservé entre le canal de forme hélicoïdale (16) et la paroi dudit premier cylindre (2) et une distance (29) étant conservée entre la partie en forme d'entonnoir (22) et le fond (8) de l'unité d'entrée par le fait que ledite secor de bride rainurée (21) vient buter contre un épaulement (9) du second cylindre, ledit écran hermétique (40) étant adapté pour s'ajuster par glissement dans ledit eu de manière à refermer progressivement ledit canal de forme hélicoïdale (40) par l'avancement axial dudit écrou de réglage (36), de sorte que la longueur de la partie fermée dudit canal peut être contrôlée en vissent ou en dévissant ledit écrou de réglage.

2. Dispositif selon la revendication 1, **caractérisé en ce que** l'ajustement entre l'écran (40) de l'écrou (36) et le cylindre (2) de l'unité d'entrée (1) est plus serré que l'ajustement entre l'écran (40) et le cylindre (18) de l'unité intermédiaire (17).
